# EUROPEAN PATENT SPECIFICATION

(11) **EP 1 307 569 B1**
(45) Date of publication and mention of the grant of the patent: **10.05.2006**
(21) Application number: 01955373.4
(22) Date of filing: 21.07.2001
(51) Int. Cl.: C12N 15/67, C12N 15/18, C12N 15/17, C12N 15/13, C12N 15/58, C07K 14/61, C07K 14/62, C07K 16/00, C12N 9/72

(54) **PREPARATION OF A RECOMBINANT PROTEIN IN A PROKARYOTIC HOST CELL BY IMPROVED CODON USE**
HERSTELLUNG EINES REKOMBINANTEN PROTEINS IN PROKARYOTISCHEN WIRTSZELLEN DURCH KODONSOPTIMISIERUNG
PREPARATION D'UNE PROTEINE DE RECOMBINAISON DANS UNE CELLULE HOTE PROCARYOTIQUE EN UTILISANT UN CODON AMELIORE

(30) Priority: 27.07.2000 DE 10037111
(43) Date of publication of application: 07.05.2003
(73) Proprietor: Boehringer Ingelheim International GmbH, 55216 Ingelheim (DE)
(72) Inventor: WERNER, Rolf-Günther, 88400 Biberach (DE); BERGEMANN, Klaus, 88400 Biberach (DE); GÖTZ, Friedrich, 72076 Tübingen (DE); PESCHEL, Andreas, 72076 Tübingen (DE)
(86) International application number: PCT/EP2001/008660
(87) International publication number: WO 2002/010411

(56) References cited:
- EP-A- 0 373 365
- WO-A-00/44926
- WO-A-98/35029
- STURMFELS ANTJE ET AL: "Secretion of human growth hormone by the food-grade bacterium Staphylococcus carnosus requires a propeptide irrespective of the signal peptide used." ARCHIVES OF MICROBIOLOGY, vol. 175, no. 4, April 2001 (2001-04), pages 295-300, XP002188286 ISSN: 0302-8933
- IKEHARA M ET AL: "SYNTHESIS OF A GENE FOR HUMAN GROWTH HORMONE AND ITS EXPRESSION IN ESCHERICHIA-COLI" PROCEEDINGS OF THE NATIONAL ACADEMY OF SCIENCES OF THE UNITED STATES, vol. 81, no. 19, 1984, pages 5956-5960, XP002188287 1984 ISSN: 0027-8424
- WILLIAMS D P ET AL: "DESIGN, SYNTHESIS AND EXPRESSION OF A HUMAN INTERLEUKIN-2 GENE INCORPORATING THE CODON USAGE BIAS FOUND IN HIGHLY EXPRESSED ESCHERICHIA COLI GENES" NUCLEIC ACIDS RESEARCH, OXFORD UNIVERSITY PRESS, SURREY, GB, vol. 16, no. 22, 25 November 1988 (1988-11-25), pages 10453-10467, XP000007466 ISSN: 0305-1048
- NAKAMURA Y ET AL: "CODON USAGE TABULATED FROM INTERNATIONAL DNA SEQUENCE DATABASES: STATUS FOR THE YEAR 2000" NUCLEIC ACIDS RESEARCH, OXFORD UNIVERSITY PRESS, SURREY, GB, vol. 28, no. 1, January 2000 (2000-01), page 292 XP002941557 ISSN: 0305-1048
- GOTZ, F, ET AL: "Complete nucleotide sequence of the lipase gene from Staphylococcus" NUCLEIC ACIDS RESEARCH, vol. 13, no. 16, 26 August 1985 (1985-08-26), pages 5895-5906, XP002188288
- LILJEQVIST S ET AL: "Surface display of functional fibronectin-binding domains on Staphylococcus carnosus" FEBS LETTERS, ELSEVIER SCIENCE PUBLISHERS, AMSTERDAM, NL, vol. 446, no. 2-3, 12 March 1999 (1999-03-12), pages 299-304, XP004259369 ISSN: 0014-5793 cited in the application
- KANAYA SHIGEHIKO ET AL: "Studies of codon usage and tRNA genes of 18 unicellular organisms and quantification of Bacillus subtilis tRNAs: Gene expression level and species-specific diversity of codon usage based on multivariate analysis." GENE (AMSTERDAM), vol. 238, no. 1, pages 143-155, XP002188453 ISSN: 0378-1119
- MAKRIDES: "Strategies for Achieving High-Level Expression of Genes in Escherichia coli" MICROBIOLOGICAL REVIEWS, AMERICAN SOCIETY FOR MICROBIOLOGY, WASHINGTON, DC, US, vol. 60, no. 3, 1 September 1996 (1996-09-01), pages 512-538, XP002095235 ISSN: 0146-0749
- PSCHORR, J. ET AL.: "PRODUCTION OF THE IMMUNOGLOBULIN VARIABLE DOMAIN REIV VIA A FUSION PROTEIN SYNTHESIZED BY STAPHYLOCOCCUS CARNOSUS" BIOLOGICAL CHEMISTRY, vol. 375, April 1994 (1994-04), pages 271-280,

### SEQUENZPROTOKOLL

<110> Boehringer Ingelheim International GmbH
   <120>
   <130>
   <160> 3
   <170> PatentIn Ver. 2.1
<210> 1 (SEQ:ID-NO.1)
   <211> 1383
   <212> DNA
   <213> Homo sapiens
<400> 1
   nnagatctaa aggaggtaat tcatatgaaa gaaacaaaac atcaacacac attttctatc 60
   cgtaagtcgg cttatggtgc cgcgtcggtt atggtcgcat catgtatatt tgtcatcggt 120
   gggggcgtgg cagaggcaaa tgattcgaca acacaaacaa cgacaccact agaagtcgct 180
   caaacgtcgc agcaagaaac acatacacat caaacacctg ttacatcatt acatactgca 240
   acacctgaac atgttgatga ctctaaagaa gcaacacctt tacctgaaaa agcagagtca 300
   ccaaaaaccg aagtgacagt tcaaccttca tcgcatacac aggaagtacc tgcgttacat 360
   aaaaaaacac agcaacaacc ggcgtataag gataaaacgg taccagagtc aacgatagca 420
   tcaaagtcgg ttgaatcaaa taaagcaaca gaaaatgaga tgtcacctgt tgaacatcat 480
   gcttcaaatg tggaaaaacg tgaagataga ttggagacta atgagacaac accgccatca 540
   gtggaccgtg aatttagcca taaaatcatc aataatacgc acgtaaatcc aaaaacggat 600
   ggacaaacaa acgttaatgt tgatacgaaa acgatagaca ccgtttcacc gaaagatgac 660
   agaatagata cggcgcaacc gaaacaagtc gacgttccta aagaaaatac aacggcacaa 720
   aataaattta catcacaagc gagcgacaaa aaaccaacag taaaagatgc atcaggtggt 780
   gatgatgatg ataaatttcc aacaattccc ttaagtcgat tgttcgataa cgctatgtta 840
   cgtgcacata gattacacca gctagcattc gatacttatc aagaatttga ggaagcttac 900
   atcccaaaag aacaaaagta tagcttttta caaaatccgc aaacatcatt atgtttctct 960
   gaatcaattc caacacctag taaccgtgag gaaactcagc aaaaatcaaa tttagaactt 1020
   ttacgtatta gcttgttact tatacaatct tggttagaac cagttcaatt tttacgttca 1080
   gtattcgcaa atagtttagt ctatggtgct tcagactcta acgtatacga tttattgaaa 1140
   gacttagaag aaggaattca aacattaatg ggtcgtttgg aagatggttc accaagaact 1200
   ggccaaattt ttaaacaaac atatagcaaa ttcgatacta attcacataa cgatgacgca 1260
   ttacttaaaa attacggttt attgtattgt tttcgtaaag atatggataa agttgaaaca 1320
   ttcttacgca tagtacaatg ccgttctgtt gaaggatcat gtggttttta atgataactg 1380
   cag                                                               1383
<210> 2 (SEQ:ID-NO.2)
   <211> 448
   <212> PRT
   <213> Homo sapiens
<400> 2
   Met Lys Glu Thr Lys His Gln His Thr Phe Ser Ile Arg Lys Ser Ala
    1               5                  10                  15
   Tyr Gly Ala Ala Ser Val Met Val Ala Ser Cys Ile Phe Val Ile Gly
               20                  25                  30
   Gly Gly Val Ala Glu Ala Asn Asp Ser Thr Thr Gln Thr Thr Thr Pro
           35                  40                  45
   Leu Glu Val Ala Gln Thr Ser Gln Gln Glu Thr His Thr His Gln Thr
       50                  55                  60
   Pro Val Thr Ser Leu His Thr Ala Thr Pro Glu His Val Asp Asp Ser
   65                  70                  75                  80
   Lys Glu Ala Thr Pro Leu Pro Glu Lys Ala Glu Ser Pro Lys Thr Glu
                   85                  90                  95
   Val Thr Val Gln Pro Ser Ser His Thr Gln Glu Val Pro Ala Leu His
               100                 105                 110
   Lys Lys Thr Gln Gln Gln Pro Ala Tyr Lys Asp Lys Thr Val Pro Glu
           115                 120                 125
   Ser Thr Ile Ala Ser Lys Ser Val Glu Ser Asn Lys Ala Thr Glu Asn
       130                 135                 140
   Glu Met Ser Pro Val Glu His His Ala Ser Asn Val Glu Lys Arg Glu
   145                 150                 155                 160
   Asp Arg Leu Glu Thr Asn Glu Thr Thr Pro Pro Ser Val Asp Arg Glu
                   165                 170                 175
   Phe Ser His Lys Ile Ile Asn Asn Thr His Val Asn Pro Lys Thr Asp
               180                 185                 190
   Gly Gln Thr Asn Val Asn Val Asp Thr Lys Thr Ile Asp Thr Val Ser
           195                 200                 205
   Pro Lys Asp Asp Arg Ile Asp Thr Ala Gln Pro Lys Gln Val Asp Val
       210                 215                 220
   Pro Lys Glu Asn Thr Thr Ala Gln Asn Lys Phe Thr Ser Gln Ala Ser
   225                 230                 235                 240
   Asp Lys Lys Pro Thr Val Lys Asp Ala Ser Gly Gly Asp Asp Asp Asp
                   245                 250                 255
   Lys Phe Pro Thr Ile Pro Leu Ser Arg Leu Phe Asp Asn Ala Met Leu
               260                 265                 270
   Arg Ala His Arg Leu His Gln Leu Ala Phe Asp Thr Tyr Gln Glu Phe
           275                 280                 285
   Glu Glu Ala Tyr Ile Pro Lys Glu Gln Lys Tyr Ser Phe Leu Gln Asn
           290                 295                 300
   Pro Gln Thr Ser Leu Cys Phe Ser Glu Ser Ile Pro Thr Pro Ser Asn
   305                 310                 315                 320
   Arg Glu Glu Thr Gln Gln Lys Ser Asn Leu Glu Leu Leu Arg Ile Ser
                   325                 330                 335
   Leu Leu Leu Ile Gln Ser Trp Leu Glu Pro Val Gln Phe Leu Arg Ser
               340                 345                 350
   Val Phe Ala Asn Ser Leu Val Tyr Gly Ala Ser Asp Ser Asn Val Tyr
           355                 360                 365
   Asp Leu Leu Lys Asp Leu Glu Glu Gly Ile Gln Thr Leu Met Gly Arg
       370                 375                 380
   Leu Glu Asp Gly Ser Pro Arg Thr Gly Gln Ile Phe Lys Gln Thr Tyr
   385                 390                 395                 400
   Ser Lys Phe Asp Thr Asn Ser His Asn Asp Asp Ala Leu Leu Lys Asn
                   405                 410                 415
   Tyr Gly Leu Leu Tyr Cys Phe Arg Lys Asp Met Asp Lys Val Glu Thr
               420                 425                 430
   Phe Leu Arg Ile Val Gln Cys Arg Ser Val Glu Gly Ser Cys Gly Phe
           435                 440                 445
<210> 3 (SEQ:ID-NO.3)
   <211> 1383
   <212> DNA
   <213> Artificial Sequence
<220>
   <223> Description of Artificial Sequence: homo sapiens flanked by vectorial sequence
<400> 3
   nnagatctaa aggaggtaat tcatatgaaa gaaacaaaac atcaacacac attttctatc 60
   cgtaagtcgg cttatggtgc cgcgtcggtt atggtcgcat catgtatatt tgtcatcggt 120
   gggggcgtgg cagaggcaaa tgattcgaca acacaaacaa cgacaccact agaagtcgct 180
   caaacgtcgc agcaagaaac acatacacat caaacacctg ttacatcatt acatactgca 240
   acacctgaac atgttgatga ctctaaagaa gcaacacctt tacctgaaaa agcagagtca 300
   ccaaaaaccg aagtgacagt tcaaccttca tcgcatacac aggaagtacc tgcgttacat 360
   aaaaaaacac agcaacaacc ggcgtataag gataaaacgg taccagagtc aacgatagca 420
   tcaaagtcgg ttgaatcaaa taaagcaaca gaaaatgaga tgtcacctgt tgaacatcat 480
   gcttcaaatg tggaaaaacg tgaagataga ttggagacta atgagacaac accgccatca 540
   gtggaccgtg aatttagcca taaaatcatc aataatacgc acgtaaatcc aaaaacggat 600
   ggacaaacaa acgttaatgt tgatacgaaa acgatagaca ccgtttcacc gaaagatgac 660
   agaatagata cggcgcaacc gaaacaagtc gacgttccta aagaaaatac aacggcacaa 720
   aataaattta catcacaagc gagcgacaaa aaaccaacag taaaagatgc atcaggtggt 780
   gatgatgatg ataaatttcc aacaattccc ttaagtcgat tgttcgataa cgctatgtta 840
   cgtgcacata gattacacca gctagcattc gatacttatc aagaatttga ggaagcttac 900
   atcccaaaag aacaaaagta tagcttttta caaaatccgc aaacatcatt atgtttctct 960
   gaatcaattc caacacctag taaccgtgag gaaactcagc aaaaatcaaa tttagaactt 1020
   ttacgtatta gcttgttact tatacaatct tggttagaac cagttcaatt tttacgttca 1080
   gtattcgcaa atagtttagt ctatggtgct tcagactcta acgtatacga tttattgaaa 1140
   gacttagaag aaggaattca aacattaatg ggtcgtttgg aagatggttc accaagaact 1200
   ggccaaattt ttaaacaaac atatagcaaa ttcgatacta attcacataa cgatgacgca 1260
   ttacttaaaa attacggttt attgtattgt tttcgtaaag atatggataa agttgaaaca 1320
   ttcttacgca tagtacaatg ccgttctgtt gaaggatcat gtggttttta atgataactg 1380
   cag                                                               1383

### SEQUENZPROTOKOLL

<110> Boehringer Ingelheim International GmbH
   <120>
   <130>
   <160> 3
   <170> PatentIn Ver. 2.1
<210> 1 (SEQ ID NO:1)
   <211> 1383
   <212> DNA
   <213> Homo sapiens
<400> 1
   nnagatctaa aggaggtaat tcatatgaaa gaaacaaaac atcaacacac attttctatc 60
   cgtaagtcgg cttatggtgc cgcgtcggtt atggtcgcat catgtatatt tgtcatcggt 120
   gggggcgtgg cagaggcaaa tgattcgaca acacaaacaa cgacaccact agaagtcgct 180
   caaacgtcgc agcaagaaac acatacacat caaacacctg ttacatcatt acatactgca 240
   acacctgaac atgttgatga ctctaaagaa gcaacacctt tacctgaaaa agcagagtca 300
   ccaaaaaccg aagtgacagt tcaaccttca tcgcatacac aggaagtacc tgcgttacat 360
   aaaaaaacac agcaacaacc ggcgtataag gataaaacgg taccagagtc aacgatagca 420
   tcaaagtcgg ttgaatcaaa taaagcaaca gaaaatgaga tgtcacctgt tgaacatcat 480
   gcttcaaatg tggaaaaacg tgaagataga ttggagacta atgagacaac accgccatca 540
   gtggaccgtg aatttagcca taaaatcatc aataatacgc acgtaaatcc aaaaacggat 600
   ggacaaacaa acgttaatgt tgatacgaaa acgatagaca ccgtttcacc gaaagatgac 660
   agaatagata cggcgcaacc gaaacaagtc gacgttccta aagaaaatac aacggcacaa 720
   aataaattta catcacaagc gagcgacaaa aaaccaacag taaaagatgc atcaggtggt 780
   gatgatgatg ataaatttcc aacaattccc ttaagtcgat tgttcgataa cgctatgtta 840
   cgtgcacata gattacacca gctagcattc gatacttatc aagaatttga ggaagcttac 900
   atcccaaaag aacaaaagta tagcttttta caaaatccgc aaacatcatt atgtttctct 960
   gaatcaattc caacacctag taaccgtgag gaaactcagc aaaaatcaaa tttagaactt 1020
   ttacgtatta gcttgttact tatacaatct tggttagaac cagttcaatt tttacgttca 1080
   gtattcgcaa atagtttagt ctatggtgct tcagactcta acgtatacga tttattgaaa 1140
   gacttagaag aaggaattca aacattaatg ggtcgtttgg aagatggttc accaagaact 1200
   ggccaaattt ttaaacaaac atatagcaaa ttcgatacta attcacataa cgatgacgca 1260
   ttacttaaaa attacggttt attgtattgt tttcgtaaag atatggataa agttgaaaca 1320
   ttcttacgca tagtacaatg ccgttctgtt gaaggatcat gtggttttta atgataactg 1380
   cag                                                               1383
<210> 2 (SEQ ID NO:2)
   <211> 448
   <212> PRT
   <213> Homo sapiens
<400> 2
   Met Lys Glu Thr Lys His Gln His Thr Phe Ser Ile Arg Lys Ser Ala
   1               5                   10                  15
   Tyr Gly Ala Ala Ser Val Met Val Ala Ser Cys Ile Phe Val Ile Gly
               20                  25                  30
   Gly Gly Val Ala Glu Ala Asn Asp Ser Thr Thr Gln Thr Thr Thr Pro
           35                  40                  45
   Leu Glu Val Ala Gln Thr Ser Gln Gln Glu Thr His Thr His Gln Thr
       50                  55                  60
   Pro Val Thr Ser Leu His Thr Ala Thr Pro Glu His Val Asp Asp Ser
   65                  70                  75                  80
   Lys Glu Ala Thr Pro Leu Pro Glu Lys Ala Glu Ser Pro Lys Thr Glu
                   85                  90                  95
   Val Thr Val Gln Pro Ser Ser His Thr Gln Glu Val Pro Ala Leu His
               100                 105                 110
   Lys Lys Thr Gln Gln Gln Pro Ala Tyr Lys Asp Lys Thr Val Pro Glu
           115                 120                 125
   Ser Thr Ile Ala Ser Lys Ser Val Glu Ser Asn Lys Ala Thr Glu Asn
       130                 135                 140
   Glu Met Ser Pro Val Glu His His Ala Ser Asn Val Glu Lys Arg Glu
   145                 150                 155                 160
   Asp Arg Leu Glu Thr Asn Glu Thr Thr Pro Pro Ser Val Asp Arg Glu
                   165                 170                 175
   Phe Ser His Lys Ile Ile Asn Asn Thr His Val Asn Pro Lys Thr Asp
               180                 185                 190
   Gly Gln Thr Asn Val Asn Val Asp Thr Lys Thr Ile Asp Thr Val Ser
           195                 200                 205
   Pro Lys Asp Asp Arg Ile Asp Thr Ala Gln Pro Lys Gln Val Asp Val
       210                 215                 220
   Pro Lys Glu Asn Thr Thr Ala Gln Asn Lys Phe Thr Ser Gln Ala Ser
   225                 230                 235                 240
   Asp Lys Lys Pro Thr Val Lys Asp Ala Ser Gly Gly Asp Asp Asp Asp
                   245                 250                 255
   Lys Phe Pro Thr Ile Pro Leu Ser Arg Leu Phe Asp Asn Ala Met Leu
               260                 265                 270
   Arg Ala His Arg Leu His Gln Leu Ala Phe Asp Thr Tyr Gln Glu Phe
           275                 280                 285
   Glu Glu Ala Tyr Ile Pro Lys Glu Gln Lys Tyr Ser Phe Leu Gln Asn
       290                 295                 300
   Pro Gln Thr Ser Leu Cys Phe Ser Glu Ser Ile Pro Thr Pro Ser Asn
   305                 310                 315                 320
   Arg Glu Glu Thr Gln Gln Lys Ser Asn Leu Glu Leu Leu Arg Ile Ser
                   325                 330                 335
   Leu Leu Leu Ile Gln Ser Trp Leu Glu Pro Val Gln Phe Leu Arg Ser
               340                 345                 350
   Val Phe Ala Asn Ser Leu Val Tyr Gly Ala Ser Asp Ser Asn Val Tyr
           355                 360                 365
   Asp Leu Leu Lys Asp Leu Glu Glu Gly Ile Gln Thr Leu Met Gly Arg
   370 375 380
   Leu Glu Asp Gly Ser Pro Arg Thr Gly Gln Ile Phe Lys Gln Thr Tyr
   385 390 395 400
   Ser Lys Phe Asp Thr Asn Ser His Asn Asp Asp Ala Leu Leu Lys Asn
   405 410 415
   Tyr Gly Leu Leu Tyr Cys Phe Arg Lys Asp Met Asp Lys Val Glu Thr
   420 425 430
   Phe Leu Arg Ile Val Gln Cys Arg Ser Val Glu Gly Ser Cys Gly Phe
   435 440 445
<210> 3 (SEQ ID NO:3)
   <211> 1383
   <212> DNA
   <213> Artificial Sequence
<220>
   <223> Description of Artificial Sequence: homo sapiens flanked by vectorial sequence
<400> 3
   nnagatctaa aggaggtaat tcatatgaaa gaaacaaaac atcaacacac attttctatc 60
   cgtaagtcgg cttatggtgc cgcgtcggtt atggtcgcat catgtatatt tgtcatcggt 120
   gggggcgtgg cagaggcaaa tgattcgaca acacaaacaa cgacaccact agaagtcgct 180
   caaacgtcgc agcaagaaac acatacacat caaacacctg ttacatcatt acatactgca 240
   acacctgaac atgttgatga ctctaaagaa gcaacacctt tacctgaaaa agcagagtca 300
   ccaaaaaccg aagtgacagt tcaaccttca tcgcatacac aggaagtacc tgcgttacat 360
   aaaaaaacac agcaacaacc ggcgtataag gataaaacgg taccagagtc aacgatagca 420
   tcaaagtcgg ttgaatcaaa taaagcaaca gaaaatgaga tgtcacctgt tgaacatcat 480
   gcttcaaatg tggaaaaacg tgaagataga ttggagacta atgagacaac accgccatca 540
   gtggaccgtg aatttagcca taaaatcatc aataatacgc acgtaaatcc aaaaacggat 600
   ggacaaacaa acgttaatgt tgatacgaaa acgatagaca ccgtttcacc gaaagatgac 660
   agaatagata cggcgcaacc gaaacaagtc gacgttccta aagaaaatac aacggcacaa 720
   aataaattta catcacaagc gagcgacaaa aaaccaacag taaaagatgc atcaggtggt 780
   gatgatgatg ataaatttcc aacaattccc ttaagtcgat tgttcgataa cgctatgtta 840
   cgtgcacata gattacacca gctagcattc gatacttatc aagaatttga ggaagcttac 900
   atcccaaaag aacaaaagta tagcttttta caaaatccgc aaacatcatt atgtttctct 960
   gaatcaattc caacacctag taaccgtgag gaaactcagc aaaaatcaaa tttagaactt 1020
   ttacgtatta gcttgttact tatacaatct tggttagaac cagttcaatt tttacgttca 1080
   gtattcgcaa atagtttagt ctatggtgct tcagactcta acgtatacga tttattgaaa 1140
   gacttagaag aaggaattca aacattaatg ggtcgtttgg aagatggttc accaagaact 1200
   ggccaaattt ttaaacaaac atatagcaaa ttcgatacta attcacataa cgatgacgca 1260
   ttacttaaaa attacggttt attgtattgt tttcgtaaag atatggataa agttgaaaca 1320
   ttcttacgca tagtacaatg ccgttctgtt gaaggatcat gtggttttta atgataactg 1380
   cag 1383

## Description

### Field of the Invention:

The present invention relates to advantageous processes for preparing heterologous proteins in prokaryotic host cells by improved codon use and/or expression of tRNAs which code for rarely occurring codons in the said host cell.

### Background of the Invention:

Numerous bacteria, particularly Gram-positive bacteria, for example, have already been used as host cells for the preparation of heterologous recombinant proteins, e.g. secreted proteins, in an endotoxin-free environment (references 8, 9, 19). The bacterium *Staphylococcus carnosus* (*S. carnosus*), for example, which is used in the food industry for the fermentation of meat and fish, is a suitable bacterium for this purpose. It is free from virulence factors and proteolytic activities in the supernatant and is capable of secreting large amounts of protein (10). Moreover, only small amounts of host cell-coded proteins are secreted, which makes it easier to purify the protein produced.

Bacterial enzymes (7, 4, 17), for example, have already been recombinantly produced and expressed in *S. carnosus.* Recombinant *S. carnosus* strains which express antigens such as the Streptococcus protein G on their surface,are particularly promising as live vaccines (5, 12). For example, Liliquist et al,(5) decribes expression of three different heterologous fibronectin binding proteins.The expression vectores used for expression, comprises the promoter, signal sequence, and propeptide sequence from a *S. hyicus* lipase gene construct, wich was optimized for the expression in *S. carnosus.*

Expression of heterologous proteins of higher eucaryotics in *S. carnosus* was also described in the prior art. Pschorr et al (20), for example, have reported the expression of the immunoglobulin variable domain (REIv) in *S. carnosus.* The heterologous REIv was expressed as a fusion protein, that consists of an amminoterminal portion of the pre-pro-peptide of *S*. *hyicus* lipase, a hexahistidine affinity tag, followed by the recognition sequence of IgA protease and REIv. The pre-pro-peptide of *S. hyicus* was used for secretion of the heterologous REIv into the culture supernatant.

One crucial disadvantage of numerous bacterial systems,including *S. carnosus,* is their use of rare codons, which is very different from the codon preference in human genes. For example, the presence of rare codons in *E. coli* led to delayed and reduced expression of recombinant genes (2, 6). In this context, Ikehara et al (21) chemically synthesized a human growth hormone (hGH) gene for the expression in *E. coli,* wherein the amino acid codons used were chosen mainly from those found in a gene for elongation factor Tu of *E. coli.*

EP 373 365 describes transfection of *E. coli* with the *E. coli* genes encoding for t-RNAs which recognize the AGG and AGA codons and incorporate Arginin during the biosynthesis. Codons for AGA and AGG are normally rarely used codons in *E. coli.*

The problem of the present invention is therefore to overcome this disadvantage of the prior art and provide a prokaryotic expression system with improved properties for the expression of recombinant proteins, such as hGH, in *S. carnosus.*

### Description of the invention

The problem is solved within the scope of the claims and specification of the present invention.

The use of the singular or plural in the claims or specification is not intended to be limiting in any way and also includes the alternative form.

The invention relates to a process for preparing a LipP-hGH fusion protein in S. carnosus as described herein more in detail, characterised in that the codon use of the host cell for host cell genes is determined, in the nucleic acid coding for the heterologous recombinant protein in the host cell rarely occurring codons are replaced by frequent codons, said host cell is transformed with said nucleic acid coding for the recombinant protein and said recombinant nucleic acid is expressed. With the process described for the expression of the LipP-hGH fusion protein in S. carnosus provided herein, it is possible to achieve a significantly better expression rate of the heterologous proteins compared with the process known from the prior art.

The processes described herein are also particularly advantageous for the expression of recombinant proteins on the surface of prokaryotes, as the covalent anchoring on the cell wall depends on intact C-termini, which are absent from the secreted truncated proteins which are not prepared by the process described herein.

By heterologous is meant that said protein in said prokaryotic host cell is not native, i.e. it occurs as a protein peculiar to the host cell. "Recombinant" means produced by molecular-biological methods. A heterologous recombinant protein may be any protein known to the skilled person, such as, for example, insulin, hGH, tPA, cytokines, such as e.g. interleukins (IL) such as IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, interferon (IFN) alpha, IFN beta, IFN gamma, IFN omega or IFN tau, tumour necrosis factor (TNF), TNF alpha and TNF beta, TRAIL; G-CSF, GM-CSF, M-CSF, MCP-1 and VEGF. Prokaryotic host cells may be any host cells known to the skilled person, particularly Gram-positive and Gram-negative host cells. These may be, for example, *Escherichia coli, Bacillus subtilis, Streptomyces, Proteus mirabilis* or preferably *Staphylococcus,* such as e.g. *Staphylococcus carnosus* in particular, as available from public collections, e.g. the Deutsche Sammlung von Mikroorganismen und Zellkulturen GmbH, Braunschweig, Germany, e.g. Strain TM300 (DSM 4600, 4601 or 4602).

Within the scope of the process described herein, first of all the use of the codons, i.e. the coding nucleotide triplets, is analysed for said host cell for as many native proteins as possible in the basic coding gene sequence. The codon frequencies of 51 genes were analysed for the host organism *Staphylococcus carnosus* Strain TM300(10) by way of example and are shown in the following Table 1:

**Table 1**

| Amino acid | Codon | Frequency In *S. carnosus* (%) | Amino acid | Codon | Frequency In *S. Carnosus* (%) |
|---|---|---|---|---|---|
| A (Ala) | GCA | 49.0 | P (Pro) | CCA | 43.5 |
| | GCT | 32.0 | | CCT | 31.3 |
| | GCG | 12.3 | | CCG | 23.0 |
| | GCC | 6.7 | | CCC | 2.2 |
| C (Cys) | TGT | 72.7 | Q (Gln) | CAA | 89.4 |
| | TGC | 27.3 | | CAG | 10.6 |
| D (Asp) | GAT | 79.4 | R (Arg) | CGT | 54.9 |
| | GAC | 20.6 | | AGA | 20.9 |
| E (Glu) | GAA | 90.4 | | CGC | 12.2 |
| | GAG | 9.6 | | CGA | 10.3 |
| F (Phe) | TTT | 53.7 | | AGG | 0.8 |
| | TTC | 46.3 | | CGG | 0.8 |
| G (Gly) | GGT | 53.9 | S (Ser) | TCA | 33.9 |
| | GGA | 22.6 | | TCT | 27.7 |
| | GGC | 16.4 | | AGT | 21.0 |
| | GGG | 7.1 | | AGC | 12.2 |
| H (His) | CAT | 75.8 | | TCC | 2.5 |
| | CAC | 24.2 | | TCG | 2.7 |
| I (Ile) | ATT | 61.7 | T (Thr) | ACA | 52.6 |
| | ATC | 27.6 | | ACT | 32.8 |
| | ATA | 10.7 | | ACG | 10.5 |
| K (Lys) | AAA | 88.1 | | ACC | 4.1 |
| | AAG | 11.9 | V (Val) | GTT | 36.7 |
| L (Leu) | TTA | 57.2 | | GTA | 36.7 |
| | TTG | 18.8 | | GTG | 13.6 |
| | CTT | 12.0 | | GTC | 12.9 |
| | CTA | 4.9 | W (Trp) | TGG | 100 |
| | CTG | 5.6 | Y (Tyr) | TAT | 76.4 |
| | CTC | 1.5 | | TAC | 23.6 |
| M (Met) | ATG | 100 | * (Stop) | TAA | 68.6 |
| N (Asn) | AAT | 64.9 | | TAG | 19.6 |
| | AAC | 35.1 | | TGA | 11.8 |

It was found that coding sequences of *Staphylococci* for example have a very low G+C content of about 30% and the A+T-rich codons are preferred. Therefore according to the invention the production of hGH, is increased in two ways: (i) exchanging rare codons in the coding gene sequence for frequent ones (cf. also Example 1) and/or (ii) expressing rare tRNAs (tRNA = transfer RNA; cf. also Example 2).

The present patent specification also describes processes for preparing a heterologous recombinant protein, wherein for *S. carnosus,* as shown in Table 1, or in similar manner for every other host cell described herein, either some or all of the rarely occurring codons are replaced by frequent ones, for example according to the analysis in Table 1 the Glu codon GAG is replaced by GAA or the Leu-codons CTC, CTG and CTA are replaced by TTA, TTG or CTT.

The present patent specification further describes a process for preparing a heterologous recombinant protein in a prokaryotic host cell, characterised in that the codon use of the host cell for host cell genes is determined, said host cell with the nucleic acid which codes tRNA or tRNAs specific for rarely occurring codons is transformed with said nucleic acid coding for the recombinant protein, and said recombinant nucleic acid is expressed (cf. also Example 2). The skilled person can determine the rare codons of the host cell by analysing the genes or the cDNAs (or by reverse transcription of the mRNA). This has been done, for example, for the host cell *S*. *carnosus* Strain TM300 (10) in Table 1. The process described herein includes the introduction of tRNAs or nucleic acid coding therefore, for one to all the rare tRNAs in the host cell. Because the host cell is equipped with tRNAs which are specific for rare codons, the heterologous recombinant protein according to the invention is expressed in larger amounts than is the case in known processes from the prior art. This surprisingly advantageous property of the process described herein is also shown in Example 2. This may occur either on its own or in conjunction with the substitution of frequent codons for codons which are rare for the host cell in the nucleic acid coding for the heterologous protein.

Therefore the present patent specification describes a process which is characterised in that, in the nucleic acid coding for the heterologous recombinant protein in the host cell, rarely occurring codons are replaced by frequent codons and said host cell is transformed with tRNA or tRNAs coding for rarely occurring codons and with said nucleic acid coding for the recombinant protein.

The present patent specification describes a process which is characterised in that codons which are used in the host cell with an average frequency of less than 15% are replaced by codons with an average frequency of at least 15%; preferably characterised in that codons which are used in the host cell with an average frequency of 7 to 12% are replaced by codons with an average frequency of more than 12%; most preferably characterised in that codons which are used in the host cell with an average frequency of up to 7 or 10% are replaced by codons with an average frequency of more than 7% or more than 10%. Another preferred embodiment described herein is a process which is characterised in that the codon or codons which occur least often in said host cell is replaced by the codon or codons which occur most frequently in said host cell. One exception may be if said codons are absolutely essential for the expression.

The invention as described herein is directed to a process which is characterised in that in the coding nucleic acid codons which are used in S. carnosus with an average frequency of less than 10% are replaced by codons with an average frequency of at least 10%.

The process described herein is applicable to *Escherichia coli,* as available from public collections, e.g. the Deutsche Sammlung von Mikroorganismen und Zellkulturen GmbH, Braunschweig, Germany, e.g. E.coli Strain K12 JM107 (DSM 3950). The process described herein is also applicable to a host cell selected from the genus *Staphylococcus,* preferably to *Staphylococcus carnosus* (cf*.* also Examples 1 and 2).

The process described herein is also applicable to the production of a recombinant protein, such as an antibody protein. By antibody protein is also meant a fragment, e.g. a Fab fragment (in English "Fragment antigen-binding = Fab"), a F(ab')₂ fragment, an Fv fragment (in English: "fragment variable" = fragment of the variable part) or a single-chain Fv (scFv)), minibodies, dia-, tria- and tetrabodies. The recombinant protein can also be for example, insulin, tissue plasminogen activator (tPa), or a human protein, a growth hormone such as human growth hormone (hGH, cf. Examples 1 and 2).

Another process decribed herein is characterised in that G- or C-rich codons are replaced by codons containing A or T. This means that codons which contain more G or C are replaced by codons which now contain, for example, an A or a T instead of a G or C; it does not mean that the codons must now contain exclusively G or C (cf. the most preferred embodiments hereinafter).

In the process described herein all the rare codons found may be replaced by frequent ones (cf. e.g. Table 1) and not only those specified in the preferred embodiments given hereinafter.

Another process described herein is characterised in that the codon GCC is replaced by GCA.

Another process described herein is characterised in that the codon TGC is replaced by TGT.

Another process described herein is characterised in that the codon GAC is replaced by GAT.

Another process described herein is characterised in that the codon GAG is replaced by GAA.

Another process described herein is characterised in that the codon TTC is replaced by TTT.

Another process described herein is characterised in that the codon GGG is replaced by GGT.

Another process described herein is characterised in that the codon CAC is replaced by CAT.

Another process described herein is characterised in that the codon ATA is replaced by ATT.

Another process described herein is characterised in that the codon AAG is replaced by AAA.

Another process described herein is characterised in that the codon CTC is replaced by TTA.

Another process described herein is characterised in that the codon AAC is replaced by AAT.

Another process described herein is characterised in that the codon CCC is replaced by CCA.

Another process described herein is characterised in that the codon CAG is replaced by CAA.

Another process described herein is characterised in that the codon CGG is replaced by CGT.

Another process described herein is characterised in that the codon TCG is replaced by TCA.

Another process described herein is characterised in that the codon ACC is replaced by ACA.

Another process described herein is characterised in that the codon GTC is replaced by GTT.

Another process described herein is characterised in that the codon TAC is replaced by TAT.

Another process described herein is characterised in that the codon TGA is replaced by TAA.

Yet another process described herein is characterised in that the host cell is transformed with the nucleic acid coding for AGG-tRNA or AGA-tRNA and with said nucleic acid coding for the recombinant protein.

The present invention further relates to a nucleic acid molecule coding for a lipP-hGH fusion protein comprising the sequence of SEQ ID NO:3.

### Example 1: Construction of a synthetic hGH gene with S. carnosus-specific codon use

As an example of a protein, human growth hormone (hGH) was expressed in *S. carnosus* Strain TM300.

Comparison of the codon use of the mature hGH with that shown in *S. carnosus* showed that 51 of the codons of the mature hGH, which represent 27% of the total number of codons, make up only 7% or less of the S. *carnosus* codon frequency (Tab. 2). An enterokinase cutting site was introduced at the 5' end of the DNA sequence of the mature hGH and the hGH was synthesised with *S. carnosus-specific* codon use. The codons in the synthetic gene were used in about the same frequency as in the *S. carnosus* gene. Codons which were used by *S. carnosus* in a frequency of less than 10% were not used, apart from those which were needed to introduce restriction cutting sites (cf. Tab. 2).

**Table 2 Number of codons in hGH cDNA and in the synthetic hGH DNA according to the invention**

| Amino Acid | Codon | ¹ | ² | Amino acid | Codon | ¹ | ² |
|---|---|---|---|---|---|---|---|
| A (Ala) | GCA | 1 | 4 | P (Pro) | CCA | 2 | 5 |
| | GCT | 1 | 3 | | CCT | 0 | 1 |
| | GCG | 0 | 0 | | CCG | 1 | 1 |
| | GCC | 5 | 0 | | CCC | 5 | 1 |
| C (Cys) | TGT | 2 | 3 | Q (Gln) | CAA | 2 | 11 |
| | TGC | 2 | 1 | | CAG | 11 | 2 |
| D (Asp) | GAT | 2 | 8 | R (Arg) | CGT | 1 | 7 |
| | GAC | 9 | 3 | | AGA | 0 | 2 |
| E (Glu) | GAA | 6 | 12 | | CGC | 4 | 1 |
| | GAG | 8 | 2 | | CGA | 0 | 1 |
| F (Phe) | TTT | 4 | 7 | | AGG | 5 | 0 |
| | TTC | 9 | 6 | | CGG | 1 | 0 |
| G (Gly) | GGT | 0 | 5 | S (Ser) | TCA | 3 | 8 |
| | GGA | 0 | 2 | | TCT | 3 | 4 |
| | GGC | 5 | 1 | | AGT | 1 | 3 |
| | GGG | 3 | 0 | | AGC | 6 | 3 |
| H (His) | CAT | 1 | 2 | | TCC | 4 | 0 |
| | CAC | 2 | 1 | | TCG | 1 | 0 |
| I (I1e) | ATT | 2 | 5 | T (Thr) | ACA | 5 | 6 |
| | ATC | 6 | 1 | | ACT | 1 | 4 |
| | ATA | 0 | 2 | | ACG | 1 | 0 |
| K (Lys) | AAA | 1 | 8 | | ACC | 3 | 0 |
| | AAG | 8 | 1 | V (Val) | GTT | 0 | 3 |
| L (Leu) | TTA | 1 | 17 | | GTA | 0 | 3 |
| | TTG | 0 | 5 | | GTG | 4 | 0 |
| | CTT | 1 | 3 | | GTC | 3 | 1 |
| | CTA | 4 | 1 | W (Trp) | TGG | 1 | 1 |
| | CTG | 12 | 0 | Y (Tyr) | TAT | 3 | 5 |
| | CTC | 8 | 0 | | TAC | 5 | 3 |
| M (Met) | ATG | 3 | 3 | * (Stop) | TAA | 0 | 2 |
| N (Asn) | AAT | 0 | 5 | | TAG | 1 | 0 |
| | AAC | 9 | 4 | | TGA | 0 | 1 |

| | | | | | | | |
|---|---|---|---|---|---|---|---|
| ¹number of codons in hGH cDNA | | | | | | | |
| ²number in synthetic hGH DNA (according to the invention) | | | | | | | |

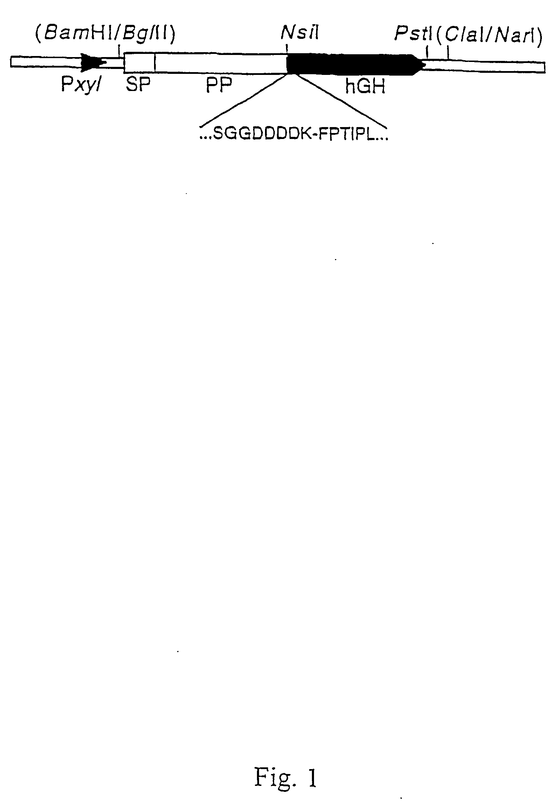

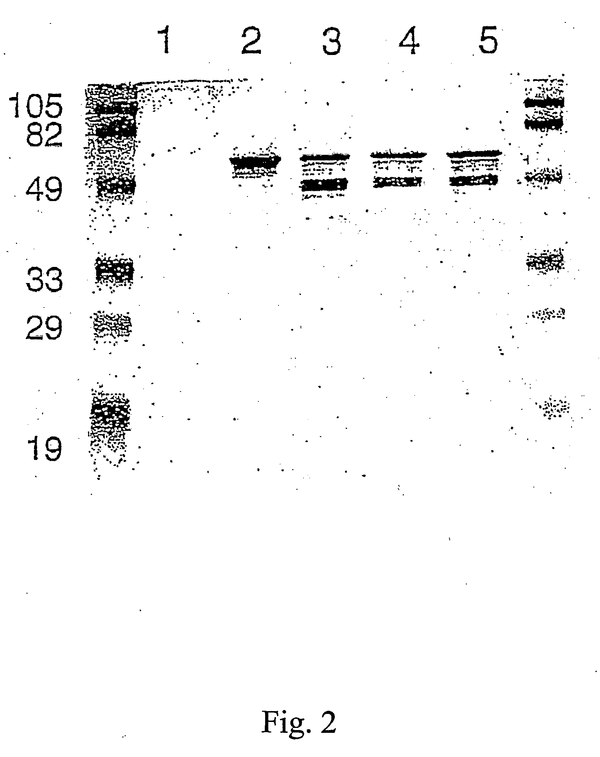

The synthetic hGH DNA sequence was synthesised from overlapping oligonucleotides which were hybridised and ligated *in vitro.* The resulting DNA fragment was cloned into the *E. coli* plasmid pSL1190 (3), verified by sequencing and inserted in the hGH expression cassette in order to replace the hGH cDNA between the NsiI and PstI cutting sites (Fig. 1). The new expression cassette was isolated as a BglII-ClaI fragment, cloned into the compatible BamHI and NarI cutting sites of the Staphylococci-expression vector pTX15 and transformed in *S. carnosus* TM300 by protoplast transformation. In the expression vector obtained, the lip-hGH fusion gene was expressed under the control of the xyl promoter of pTX15. The expression was obtained, after the inactivation of XylR, the repressor of the xyl promoter, by the addition of 0.5% xylose to the nutrient medium. The *in vitro* cloning and sequencing were carried out by standard methods (1). The expression of the hGH gene was performed as a fusion with the lip-signal and propeptide from *Staphylococcus hyicus,* which contains an enterokinase cutting site which allows the release of the mature hGH with the correct N-terminus from the fusion protein (Fig. 1). The resulting expression vector pTX-LipP-hGH4 is identical to the vector pTX-LipP-hGH2 apart from the codon use of the mature hGH. The *S. carnosus* strains containing one of the plasmids were cultivated in a modified LB medium (1% soya bean extract, 0.5% yeast extract, 0.5% NaCl) supplemented with 0.5% xylose, to activate the xyl promoter of the expression vectors. The use of the improved hGH DNA had a profound effect on the efficiency of the production of hGH in *S. carnosus.* The Lip-hGH yield was improved at least twofold and the amount of shortened Lip-hGH protein was significantly reduced (Fig. 2, traces 2 and 3). The shortened Lip-hGH proteins are not the result of extracellular proteolytic activities, as the Lip-hGH protein is stable in the *S. carnosus* supernatant. The reduced presence of the shortened proteins in the supernatant of S. *carnosus* (pTX-LipP-hGH4) shows that hGH is translated faster by the optimised DNA sequence and thus gives intracellular proteases less opportunity to cut up the protein before it is secreted away through the cytoplasmic membrane.

### Example 2: Coexpression of t-RNAs specific for rare codons in hGH cDNA

The arginine codon AGG is extremely rare in *S. carnosus* genes, as it is used in only 0.8% of the arginine codons. The corresponding tRNA genes in *S. carnosus* have not yet been identified. Therefore, we expressed E. coli tRNAs for the AGG codon in *S. carnosus.* The E. coli genes argU and argW, which code the tRNAs for the codons AGG/AGA and AGG, had been cloned with their natural promoters and terminators into the plasmids pUBS520 or pSB101 (2, 16). The fragments were isolated as SalI-SpHI (argU) and XmaI-EcoRI (argW) fragments and cloned into the corresponding restriction cutting sites of the shuttle (transfer) vector pRB572 (4). The resulting plasmids pRBargU and pRBargW were transformed in *S. carnosus* (pTX-LipP-hGH2), which express hGH cDNA.

The protein patterns in the supernatant of the *S. carnosus* strains obtained are comparable with *S. carnosus* (pTX-LipP-hGH4) with increased production of the Lip-hGH protein and less truncated proteins. Therefore, the expression of rare tRNAs according to the invention has advantageous properties for the preparation of human proteins in *S. carnosus,* for example.

### Legend relating to the Figures

FIG. 1. Expression of the Lip-hGH fusion in the plasmids pTX-LipP-hGH2 or pTX-LipP-hGH4. The gene fusion is made up of the DNA sequences coding for the signal peptide (SP) and the propeptide (PP) from *Staphylococcus hyicus* lipase and the hGH part of the mature protein (in black) and is transcribed by the xyl promoter (shown as a grey triangle). The hGH cDNA or the synthetic DNA sequence was inserted between the NsiI and the PstI cutting site. The 5' end of the hGH DNA sequence was modified to code the enterokinase cutting site 'DDDDK', followed by the mature hGH sequence, as shown underneath the gene.
FIG. 2. Detection of the Lip-hGH fusion proteins in the S. *carnosus* supernatant. Equal amounts of the supernatant were separated by SDS-PAGE on Tris-glycine gels containing 15% acrylamide and then stained with Coomassie Blue. The traces 1-5 contain supernatants of S. *carnosus* strains containing the empty control vector pTX16 (15), a derivative of pTX15 (trace 1), the plasmids pTX-LipP-hGH4 with the improved hGH gene (trace 2), pTX-LipP-hGH2 with the hGH cDNA (trace 3), pTX-LipP-hGH2 plus pRBargU (trace 4), and pTX-LipP-hGH2 plus pRBargW (trace 5). Standard proteins and their molecular weights (kDa) are shown on the left-hand side. The supernatants were concentrated by precipitation with trichloroacetic acid. SDS page was carried out by standard methods from the prior art.

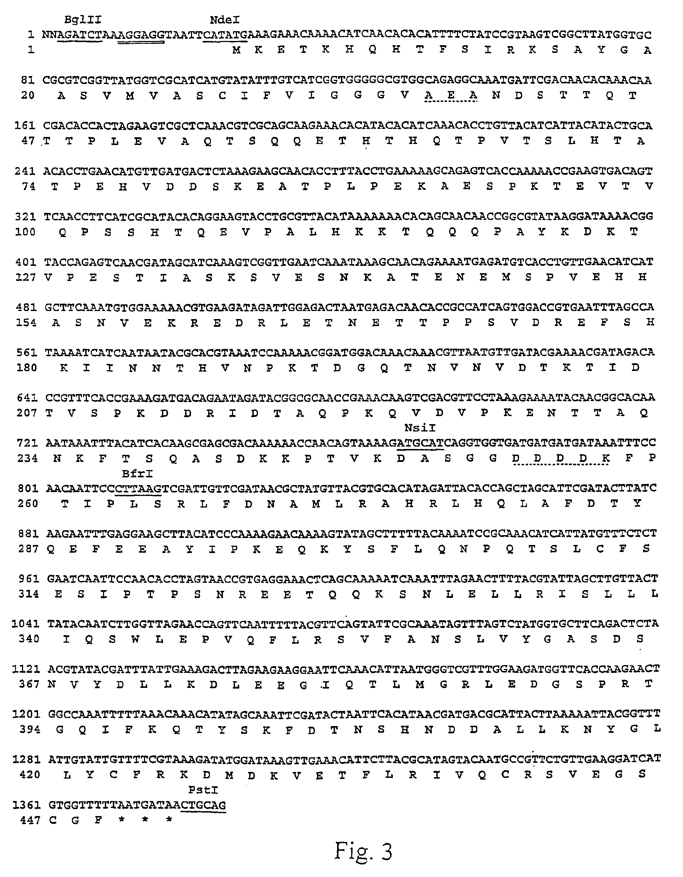

FIG. 3. Nucleotide and amino acid sequence of the hGH according to the invention (LipPhGH4, SEQID NO:3)

Relevant restriction cutting sites are underlined once, the Shine-Dalgarno sequence is underlined twice. The signal peptidase 1 and the enterokinase cleavage site in the are underlined with a broken line. Only the regions between the BglII and NdeI site and between the NsiI and the PstI site had been synthetically produced and optimised. The remainder consists of the original sequence of the signal and propeptide of the lipase from *Staphylococcus hyicus.*

### Literature

1.Ausubel, F. M., R. Brent, R. E. Kingston, D. D. Moore, J. G. Seidman, J. A. Smith, and K. Struhl. 1990. Current protocols in molecular biology. John Wiley and Sons, Inc., New York, NY.
2.Brinkmann, U., R. E. Mattes, and P. Buckel. 1989. High-level expression of recombinant genes *in Eschericia coli* is dependent on the availability of the dnaY gene product. Gene. 85:109-114.
3.Brosius, J. 1989. Superpolylinkers in cloning and expression vectors. DNA. 8:759-777.
4.Brückner, R. 1992. A series of shuttle vectors for *Bacillus subtilis* and *Escherichia coli.* Gene. 122:187-192.
5.Cano, F., S. Liljeqvist, T. N. Nguyen, P. Samuelson, J. Y. Bonnefoy, S. Stahl, and A. Robert. 1999. A surface-displayed cholera toxin B peptide improves antibody response using food-grade staphylococci for mucosal subunit vaccine delivery. FEMS Immunol. Med. Microbiol. 25:289-298.
6.Del Tito, B. J., J. M. Ward, C. J. L. Hodgson, C. Gershater, H. Edwards, L. A. Wysocki, F. A. Watson, G. Sathe, and J. F. Kane. 1995. Effects of a minor isoleucyl tRNA on heterologous protein translation in Escherichia coli. J. Bacteriol. 177:7086-7091.
7.Demleitner, G., and F. Götz. 1994. Evidence for importance of the *Staphylococcus hyicus* lipase pro-peptide in lipase secretion, stability and activity. FEMS Microbiol. Lett. 121:189-198.
8.Gilbert, M., R. Morosoli, F. Shareck, and D. Kluepfel. 1995. Production and secretion of proteins by streptomycetes. Crit. Rev. Biotechnol. 15:13-39.
9.Götz, F. 1990. Development of a cloning system in *Staphylococcus carnosus:* Different processing of the Staphylococcus hyicus lipase in Staphylococcus carnosus and *Staphylococcus hyicus,* p. 273-281. In R. P. Novick (ed.), Molecular biology of the staphylococci. VCH Publisher Inc., New York, NY.
10.Götz, F. 1990. *Staphylococcus carnosus:* a new host organism for gene cloning and protein production. J. Appl. Bacteriol. Symp. Suppl.:49S-53S.
11.Götz, F., and B. Schumacher. 1987. Improvements of protoplast transformation in *Staphylococcus carnosus.* FEMS Microbiol. Lett. 40:285-288.
12.Liljeqvist, S., F. Cano, T. N. Nguyen, M. Uhlen, A. Robert, and S. Stahl. 1999. Surface display of functional fibronectin-binding domains on *Staphylococcus carnosus.* FEBS Lett. 446:299-304.
13.Makrides, S. C. 1996. Strategies for achieving high-level expression of genes in *Escherichia coli.* Microbiol. Reviews. 60:512-538.
14.Nikoleit, K., R. Rosenstein, H. M. Verheij, and F. Götz. 1995. Comparative biochemical and molecular analysis of the *Staphylococcus hyicus, Staphylococcus aureus* and a hybrid lipase. Eur. J. Biochem. 228:732-738.
15.Peschel, A., B. Ottenwälder, and F. Götz. 1996. Inducible production and cellular location of the epidermin biosynthetic enzyme EpiB using an improved staphylococcal expression system. FEMS Microbiol. Lett. 137:279-284.
16.Schenk, P. M., S. Baumann, R. Mattes, and H. H. Steinbiss. 1995. Improved high level expression system for eucaryotic genes in *Escherichia coli* using T7 RNA polymerase and rare Arg-tRNAs.
17.Thumm, G., and F. Götz. 1997. Studies on prolysostaphin processing and characterization of the lysostaphin immunity factor (Lif) of *Staphylococcus simulans biovar staphylolyticus.* Mol. Microbiol. 23:1251-1265.
18.Vance, M. L., and N. Mauras. 1999. Growth hormone therapy in adults and children. New Engl. J. Med. 341:1206-1216.
19.Wong, S. L. 1995. Advances in the use of *Bacillus subtilis* for the expression and secretion of heterologous proteins. Curr. Opin. Biotechnol. 6:517-522_
20.Pschorr, J., Bieseler B., and Fritz H.-J. 1994. Production of the immunoglobulin variable domain REIv via a fusion protein synthesized and secreted by Staphylococcus camosus. Biol. Chem.Hoppe-Seyler 375:271-280.
21.Ikrhara M, Ohtsuka T., TokunagaY., et al. 1984. Synthesis of a gene for human growth hormone and its expression in Escherichia coli. Proc. Natl. Acad.Sci, USA 81:5956-5960.

## Claims

1. Process for expressing a LipP-hgH fusion gene in *Staphylococcus carnosus,* wherein a human Growth hormone- (hGH) gene is fused to the coding sequence of the lip-signal and propeptide of *Staphylococcus hyicus* lipase (LipP), and which comprises an enterokinase cutting site between LipP and hGH which allows the release of the mature hGH with the correct N-terminus, and wherein codons coding for the hGH gene which are used in *Staphylococcus carnosus* with an average frequence of less than 10% are replaced by codons with an average frequency of at least 10% and/or wherein said *Staphylococcus carnosus* host cell is transformed with tRNA, tRNAs or nucleic acid coding thereof, coding for codons which are used with an average frequence of less than 10%.

2. Process according to claim 1, **characterised in that** G- or C-rich codons of hGH are replaced by codons containing A or T.

3. Process according to claim 1 or 2, **characterised in that** *Staphylococcus carnosus* is transformed with the nucleic acid coding for AGG-tRNA or AGA-tRNA.

4. Process according to any of claims 1 to 3, **characterised in that** *Staphylococcus carnosus* is transformed with the nucleic acid of the E.coli genes argU and argW, which code the tRNAs for the codons AGG/AGA and AGG.

5. Process according to any of claims 1 to 4, wherein the LipP-hGH fusion protein is coded by a nucleic acid molecule comprising the sequence of SEQ:ID-NO.3.

6. Nucleic acid molecule coding for a LipP-hgG fusion protein comprising the sequence of SEQ:ID-NO.3.

## Patentansprüche

1. Verfahren zur Expression eines LipP-hGH-Fusionsgens in *Staphylococcus carnosus,* in dem ein Human-Wachstumshormon (hGH)-Gen an die codierende Sequenz des lip-Signal- und Propeptids von *Staphylococcus* hyicus-Lipase (LipP) fusioniert ist und das eine Enterokinase-Schnittstelle zwischen LipP und hGH umfasst, welche die Freisetzung des reifen hGH mit dem korrekten N-Terminus ermöglicht, und in dem Codons, die für das hGH-Gen codieren und in *Staphylococcus carnosus* mit einer durchschnittlichen Häufigkeit von weniger als 10 % verwendet werden, durch Codons mit einer durchschnittlichen Häufigkeit von mindestens 10 % ersetzt sind und/oder in dem die *Staphylococcus* carnosus-Wirtszelle mit tRNA, tRNAs oder dafür codierender Nucleinsäure transformiert ist, welche für Codons codieren, die mit einer durchschnittlichen Häufigkeit von weniger als 10 % verwendet werden.

2. Verfahren nach Anspruch 1, **dadurch gekennzeichnet, dass** die G- oder C-reichen Codons von hGH durch Codons ersetzt werden, die A oder T enthalten.

3. Verfahren nach Anspruch 1 oder 2, **dadurch gekennzeichnet, dass** *Staphylococcus carnosus* mit der Nucleinsäure transformiert wird, die für AGG-tRNA oder AGA-tRNA codiert.

4. Verfahren nach irgendeinem der Ansprüche 1 bis 3, **dadurch gekennzeichnet, dass** *Staphylococcus carnosus* mit der Nucleinsäure der E. coli-Gene argU und argW transformiert wird, welche die tRNAs für die Codons AGG/AGA und AGG codieren.

5. Verfahren nach irgendeinem der Ansprüche 1 bis 4, in dem das LipP-hGH-Fusionsprotein durch ein Nucleinsäure-Molekül codiert wird, welches die Sequenz SEQ ID NO: 3 umfasst.

6. Nucleinsäure-Molekül, das für ein LipP-hGH-Fusionsprotein codiert und die Sequenz SEQ ID NO: 3 umfasst.

## Revendications

1. Procédé pour exprimer un gène de fusion LipP-hgH dans *Staphylococcus carnosus,* où un gène d'hormone de croissance humaine (hGH) est fusionné à la séquence codante du signal lip et du propeptide de la lipase de *Staphylococcus hyicus* (LipP), et qui comprend un site de coupure pour une entérokinase entre LipP et hGH qui permet la libération de la hGH mature avec l'extrémité N-terminale correcte, et dans lequel des codons codant le gène de hGH qui sont utilisés dans *Staphylococcus carnosus* avec une fréquence moyenne de moins de 10 % sont remplacés par des codons avec une fréquence moyenne d'au moins 10 % et/ou dans lequel ladite cellule hôte de *Staphylococcus carnosus* est transformée avec un ARNt, des ARNt ou un acide nucléique les codant, codant des codons qui sont utilisés avec une fréquence moyenne de moins de 10 %.

2. Procédé selon la revendication 1 **caractérisé en ce que** des codons riches en G ou C de hGH sont remplacés par des codons contenant A ou T.

3. Procédé selon la revendication 1 ou 2 **caractérisé en ce que** *Staphylococcus carnosus* est transformé avec l'acide nucléique codant AGG-ARNt ou AGA-ARNt.

4. Procédé selon l'une quelconque des revendications 1 à 3 **caractérisé en ce que** *Staphylococcus carnosus* est transformé avec l'acide nucléique des gènes argU et argW de E. coli qui codent les ARNt pour les codons AGG/AGA et AGG.

5. Procédé selon l'une quelconque des revendications 1 à 4 dans lequel la protéine de fusion LipP-hGH est codée par une molécule d'acide nucléique comprenant la séquence de SEQ : ID-NO.3.

6. Molécule d'acide nucléique codant une protéine de fusion LipP-hgG comprenant la séquence de SEQ : ID-NO.3.
